# EUROPÄISCHE PATENTSCHRIFT

(11) **EP 0 983 377 B1**
(45) Veröffentlichungstag und Bekanntmachung des Hinweises auf die Patenterteilung: **18.05.2005**
(21) Anmeldenummer: 99908954.3
(22) Anmeldetag: 10.03.1999
(51) Int. Cl.: C12Q 1/00

(54) **Verfahren zum Nachweis von Umweltverschmutzung unter Verwendung von 14-3-3 Proteinen als Biomarker**
Method for detecting environmental pollution using 14-3-3 proteins as biomarkers
Procedé de détection de pollution de l'environnement utilisant des proteines 14-3-3 comme biomarqueurs

(30) Priorität: 12.03.1998 DE 19810721
(43) Veröffentlichungstag der Anmeldung: 08.03.2000
(60) Teilanmeldung: 04029992.7
(73) Patentinhaber: MÜLLER, Werner E. G., 65203 Wiesbaden (DE); Schröder, Heinz C., 65189 Wiesbaden (DE)
(72) Erfinder: MÜLLER, Werner E. G., 65203 Wiesbaden (DE); Schröder, Heinz C., 65189 Wiesbaden (DE)
(74) Vertreter: Krauss, Jan
(86) Internationale Anmeldenummer: PCT/EP1999/001545
(87) Internationale Veröffentlichungsnummer: WO 1999/046401

(56) Entgegenhaltungen:
- WO-A-97/33601
- WO-A-97/38315
- LEE KH, HARRINGTON MG: "14-3-3 and BSE" VETERINARY RECORD, Bd. 140, Nr. 8, 22. Februar 1997 (1997-02-22), Seiten 206-207, XP002110628 in der Anmeldung erwähnt
- ZERR I, BODEMER M, GEFELLER O, OTTO M, POSER S, WILTFANG J, WINDL O, KRETZSCHMAR HA, WEBER T: "Detection of 14-3-3 protein in the cerebrospinal fluid supports the diagnosis of Creutzfeldt-Jakob disease" ANNALS OF NEUROLOGY, Bd. 43, Nr. 1, Januar 1998 (1998-01), Seiten 32-40, XP002110629 in der Anmeldung erwähnt
- MUSLIN A J ET AL: "Interaction of 14-3-3 with signaling proteins is mediated by the recognition of phosphoserine" CELL,US,CELL PRESS, CAMBRIDGE, NA, Bd. 84, Seite 889-897 XP002089347 ISSN: 0092-8674 in der Anmeldung erwähnt
- J ZHA, H HARADA, E YANG, J JOCKEL, S J KORSMEYER: "Serine Phosphorylation of Death Agonist BAD in Response to Survival Factor Results in Binding to 14-3-3 Not BCL-XL" CELL, Bd. 87, 15. November 1996 (1996-11-15), Seiten 619-628, XP002110634 in der Anmeldung erwähnt
- R ALAM, N HACHIYA, M SAKAGUCHI, S KAWABATA, S IWANAGA, M KITAJIMA, K MIHARA, T OMURA: "cDNA Cloning and Characterization of Mitochondrial Import Stimulation Factor (MSF) Purified from Rat Liver Cytosol" JOURNAL OF BIOCHEMISTRY, Bd. 116, Nr. 2, 1994, Seiten 416-425, XP002110635 in der Anmeldung erwähnt
- WIENS, MATTHIAS; KOZIOL, CLAUDIA; HASSANEIN, HAMDY M. A.; BATEL, RENATO; SCHROEDER, HEINZ C.; MUELLER, WERNER E. G.: "Induction of gene expression of the chaperones 14-3-3 and HSP70 by PCB 118 (2,3',4,4'5-pentachlorobiphenyl)in the marine sponge Geodia cydonium: Novel biomarkers for polychlorinated biphenyls" MARINE ECOLOGY PROGRESS SERIES, Bd. 165, Nr. 0, 7. Mai 1998 (1998-05-07), Seiten 247-257, XP002110630
- MORRIS C A; NICOLAUS B; KILLE P; HARWOOD J L: "Genetic mechanisms involved in the adaptation of marine algae to heavy metal pollution" BIOCHEMICAL SOCIETY TRANSACTIONS, Bd. 26, Nr. 2, Mai 1998 (1998-05), Seite S153 XP002110631
- JUENGEL J L; MELNER M H; CLAPPER J A; TURZILLO A M; MOSS G E; NETT T M; NISWENDER G D: "Steady-state concentrations of mRNA encoding two inhibitors of protein kinase C in ovine luteal tissue" JOURNAL OF REPRODUCTION AND FERTILITY, Bd. 113, Nr. 2, Juli 1998 (1998-07), Seiten 299-305, XP002110632
- A AITKEN, D B COLLINGE, B P H VAN HEUSDEN, T ISOBE, P H ROSEBOOM, G ROSENFELD, J SOLL: "14-3-3 proteins: a highly conserved, widespread family of eukaryotic proteins" TRENDS IN BIOCHEMICAL SCIENCES, Bd. 17, Dezember 1992 (1992-12), Seiten 498-501, XP002110633 in der Anmeldung erwähnt

## Beschreibung

Die vorliegende Erfindung betrifft die Anwendung des neuen Biomarkers 14-3-3 Protein(e) zur Detektion der Belastung von Süssgewässern, Meerwasser, Trinkwasser oder Bodenproben mit natürlichen und anthropogenen Umweltchemikalien, insbesondere polychlorierten Biphenylen (PCBs) und/oder Ostrogenen/Xenoöstrogenen [(Xeno) Ostrogenen]

### 1. Beschreibung der Methode

### 1.1. Prinzip

### 1.1.1 Induktion der Expression von 14-3-3 Protein nach Einfluss von polychlorierten Biphenvien (PCBs) und (Xeno) Ostrogenen)

Von den Erfindern wurde gezeigt, daß ein neuartiges "lenkendes" Chaperon, 14-3-3 Protein(e), bei niederen aquatischen Invertebraten nach Einfluß von polychlorierten Biphenylen (PCBs) exprimiert wird. Somit war ein neuer Biomarker für PCB zumindest für aquatische Invertebraten gefunden; die bisherigen Verfahren, PCBs bei Invertebraten nachzuweisen, sind unzulänglich. Weiterhin wurde gefunden, daß die Expression von 14-3-3 Protein auch durch (Xeno) Östrogene induzierbar ist. Der Biomarker 14-3-3 Protein wurde zum Zwecke eines Effektmonitorings von PCB und von (Xeno) Östrogenen mit der Süsswasser-Muschel *Corbicula fluminea*, der Nordsee-Kliesche *Limanda limanda* sowie dem Mittelmeer-Schwamm *Suberites domuncula* als Bioindikator eingesetzt.

### 1.1.2 ELISA-Verfahren zur Quantifizierung von 14-3-3 Protein

Unsere Untersuchungen zeigten, daß sich ein ELISA zur Quantifizierung der 14-3-3 Proteine mit Rohextrakten aus Tieren nicht empfindlich genug aufbauen läßt. Deshalb haben wir einen ELISA entwickelt, der ein Anreicherungsverfahren voranstellt Es ist bekannt, daß 14-3-3 Proteine an die phosphorylierte Erkennungssequenz RS [Phosphorylierungsstelle] xSxP bindet (Muslin et al., Cell 84, 889-897,1996). Dazu wurde die Erkennungssequenz RS [P] xSxP synthetisch mit einem Biotin-Terminus hergestellt. Diese wurde in die Wells von ELISA-Platten, die mit Streptavidin beschichtet waren, gegeben. Nach Bindung von RS [P] xSxP über Biotin und Streptavidin wurden > 100 µl von Extrakten aus Tieren in die Wells gegeben. Nach Waschen wurden die RS [P] xSxP-14-3-3 Komplexe über markierte Antikörper quantifiziert. Als Markierung kann zum Beispiel die alkalische Phosphatase verwendet werden ; als Substrat zum Nachweis dient dann 5-Brom-4 Chlor-3-lndolylphosphat/Nitroblau-Tetrazolium.

### 1.1.3 Anwendung

Das ELISA-Verfahren kann sowohl in Felduntersuchungen als auch in Laboruntersuchungen (Hälterungsexperimenten) zum Biomonitoring (z. B. zur Überwachung kommunaler und industrieller Klärwerkszu- und abläufe, Flußwässer, Meerwasser) unter Benutzung geeigneter Bioindikatoren angewandt werden. Untersucht werden die Wässer in Hälterungsexperimenten nach Direktinkubationen. Bei potentiell schwachen Effekten, z. B. bei Flußwässern, können die zu untersuchenden Wässer auch aufkonzentriert werden.

### 1.2. Grundlagen

### 1.2.1 Umweltmonitoring : Biomarker und Bioindikatoren

Intensive Untersuchungen im Rahmen des Umweltmonitorings beschäftigen sich mit der Detektion von Rückständen, speziell von halogenierten Kohlenwasserstoffen und Schwermetallen. Die Ergebnisse zeigen, daß eine große Lücke bezüglich der quantitativen Abschätzung des Aussetzungsrisikos gegenüber chemischen Agenzien in der Umwelt existiert. Das Expositionsrisiko kann nicht einfach dadurch quantifiziert werden, daß die Konzentration der Xenobiotika in Geweben bestimmt wird. Viele Umweltchemikalien zeichnen sich dadurch aus, daß sie entweder nicht in Geweben des ausgewählten Bioindikators akkumulieren oder schnell metabolisiert werden. Aber auch für persistierende Xenobiotika stellt eine chemische Bestimmung lediglich eine Momentaufnahme dar, die häufig nur wenig Auskunft über das Muster der Exposition gibt, welches zur aktuellen Konzentration der Umweltchemikalie geführt hat. Weiterhin ist die Beziehung zwischen Gewebekonzentration und einer ggfs. toxischen Antwort komplex und meist noch unverstanden. Erschwerend kommt noch hinzu, daß Xenobiotika in der Umwelt als komplexe Mischungen vorliegen, die synergistische oder auch antagonistische Wechselwirkungen zeigen können.

Ein informativer Weg zur quantitativen Erfassung des Expositionsrisikos gegenüber (einer) Umweltchemikalie (n) und des daraus entstehenden potentiellen Einflusses auf den Organismus ist der Einsatz von Biomarkern bei ausgewählten Bioindikatoren.

### 1.2.2 Polychlorierte Biphenyle

Polychlorierte Biphenyle (PCBs) wurden seit den frühen 30-iger Jahren in elektrischen Geräten, bei der Herstellung von Farbstoffen, Plastikwaren, Schutzmaterialien sowie von Pestiziden, Feuerlöschmitteln und organischen Verdünnern benutzt. Sowohl diese weite Verbreitung als auch die nicht sachgerechte Entsorgung haben dazu geführt, daß PCBs ubiquitär in der Umwelt zu finden sind. Seit Mitte der 80-iger Jahre ist die Herstellung der PCBs in den USA und in Westeuropa verboten. Trotzdem sind PCBs aufgrund ihrer starken Bioakkumulation (Abramowicz, Environ. Health Perspect. 103,97-99,1995) und geringen Biotransformation (Jedamski-Grymlas et al ; Ecotoxicol. Environm. Saf. 31, 49-56,1995) auch heute noch in grossem Masse in unserer Umwelt vorhanden. PCBs lassen sich in der Luft, im Boden, in Sedimenten, in marinen Gewässern und auch in Süssgewässern nachweisen ; sie sind in der Nahrungskette -Fisch, Fleisch von Wildtieren- und im menschlichen Fettgewebe aufzufinden (Voogt et al., Int. J. Environ. Anal. Chem. 40,1-46,1990).

Der umweltbiologisch bedeutsame und molekularbiologisch interessante Effekt einiger PCBs ist ihr stimulierender Einfluß auf den Aryl-Kohlenwasserstoff Rezeptor (Ah-Rezeptor) (Ahlborg et al., Nord 1992,26. Copenhagen : Nordic Council of Ministers, 1992), der als Liganden-induzierbarer Transkriptionsfaktor fungiert (Safe, Pharmacol. Ther. 67,247-281,1995). Daran binden neben PCBs auch andere Xenobiotika, wie Dioxine und Dibenzofurane, die auch als Kontaminationen der PCBs vorkommen. Unter den Proteinen, die durch PCBs induziert werden, sind insbesondere die Cytochrom P-450 Proteine (CYP) zu nennen (Shane et al., Mut. Res. 377,1-11,1997). Deshalb wurde die Induzierbarkeit der CYP-Gene als Mass für eine Belastung mit PCBs herangezogen ; z. B. wurde die CYP1A-Expression in enzymatischen Ansätzen (Dehnen et al., Anal. Biochem. 53,373-383,1973), über immunologische Detektionsverfahren (Williams und Buhler, Biochem. Pharmacol. 33,3742-3753,1984), und/oder durch Quantifizierung der entsprechenden mRNAs (Heilman et al., DNA 7,379-387,1988) bestimmt. Es ist etabliert, daß das Maß der CYPIA-Induzierbarkeit eine relativ verläßliche Aussage über eine PCB-Belastung bei Fischen zuläßt (Stegeman et al., in : Huggett et al. (eds), Biomarkers : biochemical, physiological and histological markers of anthropogenic stress. Boca Raton, FL : Lewis, S. 235-335,1992).

Jedoch gelten die CYP-verwandten Enzyme bei Invertebraten, wie bei *Mytilus edulis,* nicht als zuverlässige Biomarker für PCBs, da in diesen Organismen sowohl die entsprechenden Proteine als auch deren kodierende Gene kaum untersucht sind (Livingstone, Mar. Ecol. Prog. Ser. 46,91-100,1988). Deshalb ist die Entwicklung neuer Biomarker für PCBs bei Invertebraten angezeigt.

PCBs werden aufgrund ihrer chemischen Struktur und (chemischen) Eigenschaften in verschiedene Klassen, wie non-ortho-und mono-ortho-substituierte PCBs eingeteilt. Funktionell lassen sich die PCBs in toxische und in hormonähnliche Substanzen einteilen (Toppari et al., Environ. Health Perspect. 104 [Suppl. 4], 741-803,1996). Toxizitätstests sind natürlich für PCBs bei Vertebraten wie auch bei Invertebraten vorhanden. Jedoch fehlen adäquate Testsysteme zum Nachweis einer subtoxischen Wirkung von PCBs bei Invertebraten im allgemeinen und einer (xeno) östrogenen/anti-(xeno) östrogenen Wirkung von PCBs im besonderen bei Invertebraten.

### 1.2.3 (Xeno) Ostrogene

(Xeno) Östrogene stellen Xenobiotika dar, die einen (xeno) östrogenen oder einen anti (xeno) östrogenen Effekt bei Metazoen auslösen. Hierunter nehmen die PCBs eine herausragende Stellung ein. Es ist bekannt, daß nieder-chlorierte PCBs, wie Aroclor 1221, 1232, 1242 oder 1248 (xeno) östrogene/anti-(xeno) östrogene Wirkung zeigen, während höher-chlorierte PCBs toxischer wirken (Toppari et al., Environ. Health Perspect. 104 [Suppl. 4], 741-803,1996). Bei Vertebraten steht mit Vitellogenin ein verläßlicher Biomarker zur Verfügung. Mittels Antikörper oder Gensonden läßt sich die Expression von Vitellogenin bei Vertebraten in Serum und Gewebe männlicher Tiere quantifizieren (Heppell et al., Environ. Heath Persp. 103 [Suppl. 7], 9-15,1995). Wir haben nach analogen Biomarkem bei Invertebraten gesucht; mit der Klonierung des Gens, das für das Protein 14-3-3 kodiert, waren wir erfolgreich.

### 1.2.4 14-3-3 Protein, ein neuartiges Chaperon

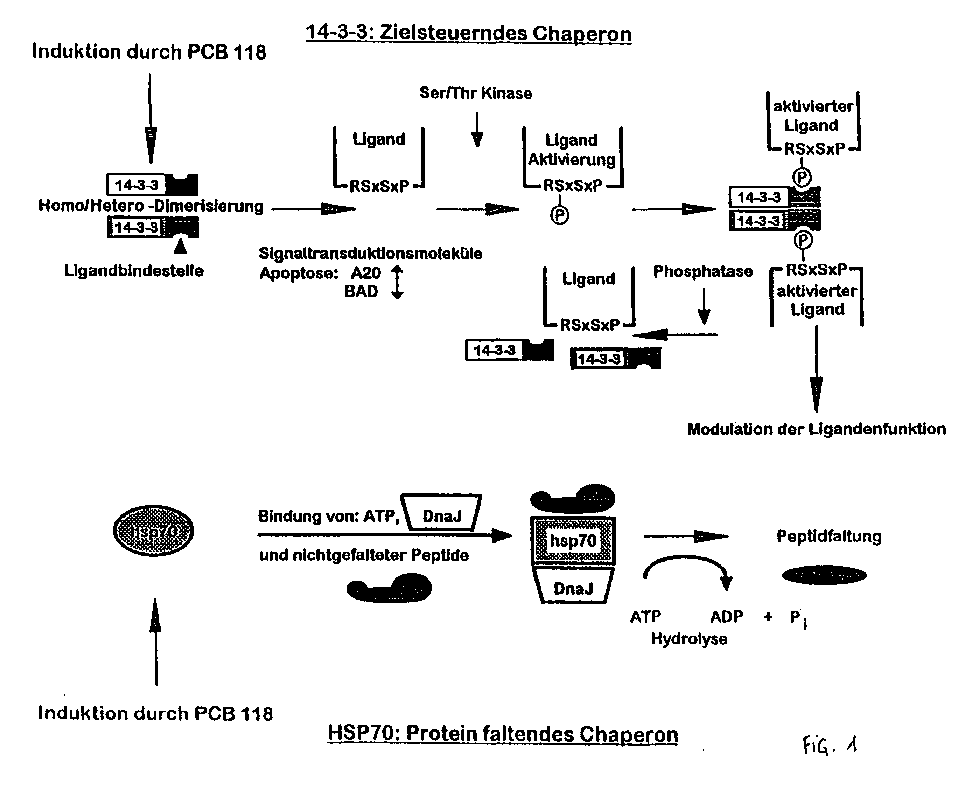

Das Hitzeschockprotein HSP70 wurde als ein Biomarker etabliert, der zur Indikation von Umweltstreß, speziell von osmotischem Streß oder einer Belastung durch Schwermetalle, dient (Koziol et al., Canad. Technical Report of Fisheries and Aquatic Sci. 2093,104-109,1996 ; Krasko et al., Aquatic Toxicol. 37,157-168,1997 ; Koziol et al., Marine Ecol. ; Progr. Ser. 154,261-268,1997). HSP70 stellt ein molekulares Chaperon dar, dessen Hauptfunktion es ist, die funktionsgerechte Faltung eines Proteins zu kontrollieren und zu erhalten (Becker und Craig, Eur. J. Biochem. 219,11-23,1994) ; FIG. 1. Im Gegensatz dazu bindet das 14-3-3 Protein (Aitken et al., Trends Biochem. Sci. 17,498-501,1992) an Funktionsmoleküle, wie (i) an Rezeptoren (z. B. dem Adrenodixon Präkursor) (Alam et al., J. Biochem. 116, 416-425,1994), (ii) an Signaltransduktions-Proteine wie Raf-1 (Muslin et al., Cell 84, 889-897,1996) oder (iii) an Schlüsselmoleküle der Apoptose (wie das BAD-Molekül oder A20) (Zha et al., Cell 87,619-628,1996) und verhindert den Transport dieser Moleküle an ihren Funktionsort ; FIG. 1. Somit stellt das 14-3-3 Protein ein neuartiges Chaperon dar, dessen Funktion es ist, an andere Proteine zu binden und diese in bestimmten Zellkompartimenten zu "fixieren". Die 14-3-3 Proteine kontrollieren essentielle Funktionen der Zelle und "immobilisieren" deren Metabolismus (Aitken et al., Trends Biochem. Sci. 17,498-501,1992). Zunächst als neuronales Protein entdeckt, gilt 14-3-3 heute als ubiquitär vorkommendes Protein, das für die genannten Prozesse aller Metazoa von entscheidender Bedeutung ist ; die Familie der 14-3-3 Proteine umfaßt sieben Isoformen (Aitken et al., Trends Biochem. Sci. 17,498-501,1992).

### 1.2.5 14-3-3 Protein : Biomarker für PCB

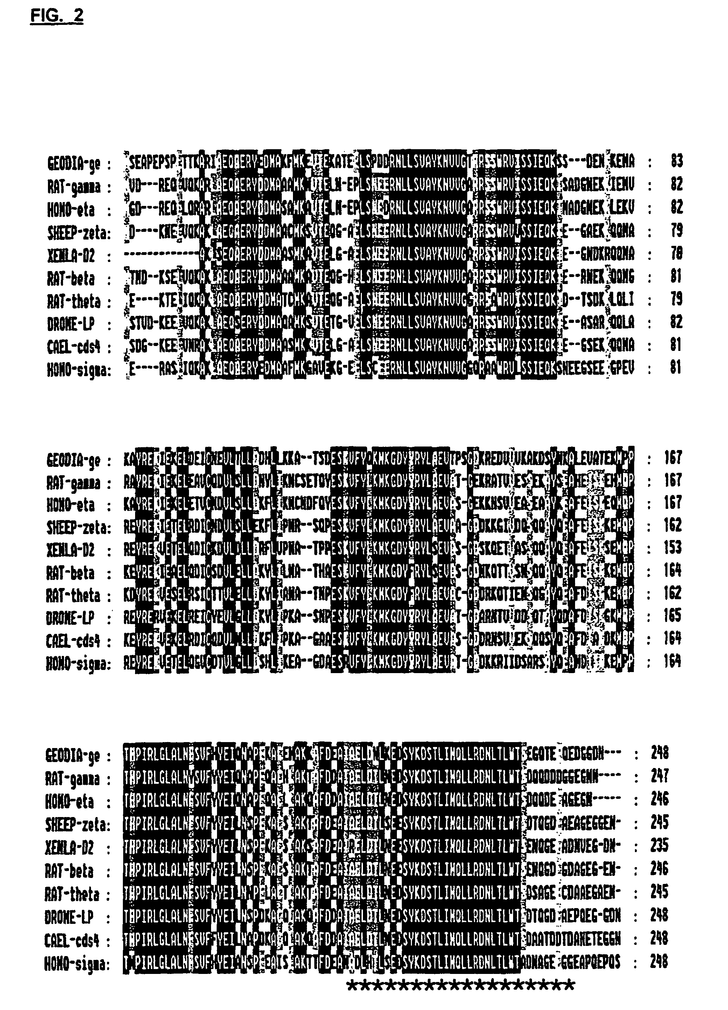

Den Antragstellern gelang es erstmalig zu zeigen, daß es sich bei den 14-3-3 Proteinen um durch PCB induzierbare Proteine -in den ersten Versuchen der Antragsteller für marine Schwämme nachgewiesen- handelt. Hierzu war zunächst die Klonierung von 14-3-3 nötig ; FIG. 2. Anschließend wurde die cDNA als Gensonde zum Nachweis der Expression auf der Ebene der mRNA und die entsprechenden Antikörper zur Bestimmung der Proteinmenge eingesetzt.

### 1.2. 614-3-3 Protein : Biomarker für (Xeno) Ostrogene

Wir konnten zeigen, daß PCB sowie 17ss-Östradiol die Expression von 14-3-3 Protein in niederen Invertebraten stark induziert. Weiter konnten wir nachweisen, daß nach Kombination beider Substanzen eine Potenzierung der Expression von 143-3 Protein eintrat.

WO 97/38318 beschreibt ein ELISA-Verfahren zur Diagnose von Prion-Erkrankungen, bei dem 14-3-3 Proteine in einer Körperflüssigkeit mittels spezifischer Antikörper bestimmt wird.

WO 97/33601 beschreibt ein immunoanalytisches Hemmverfahren zur Identifizierung von Chemikalien, die die Bindung des 14-3-3 Proteins an das pS-Raf-621 Peptid in Säugerzellen beeinflussen können, zur Auffindung und/oder der Herstellung von Pharmaka.

### 2. Beschreibung der Anwendung und des Verfahrens

### 2.1 Materialien

Die bei den nachfolgend beschriebenen Experimenten verwendeten Chemikalien wurden von folgenden Firmen bezogen : Restriktions-Endonucleasen und andere Enzyme für rekombinante DNA-Techniken und Vektoren von Stratagene (Heidelberg, Deutschland), QIAGEN (Hilden, Deutschland) und USB (Cleveland, OH, USA) ; Maisöl (C8267) von Sigma (Deisenhöfen, Deutschland) ; TRlzol Reagenz von GibcoBRL (Grand Island, NY, USA) ; CDP [Dinatrium 2-Chlor-5-(4-methoxyspiro{1,2-dioxetan-3,2'-(5'-chlor)-tricyclo[3.3.1.13,7]decan}-4-yl)phenyl-phosphat] von Boehringer Mannheim (Mannheim, Deutschland). Der polyklonale Antikörper (Kaninchen-IgG ; Kat.-Nr. PC70) gegen die konservierten Aminosäurereste der 14-3-3 Proteinfamilie und die 14-3-3 Peptide aa221 bis aa242 wurden von Calbiochem/Oncogene (Cambridge, MA, USA) erhalten. PCB118 wurde von der Dr. Ehrenstorfer GmbH (Augsburg, Deutschland) erworben.

### 2.2 Extraktgewinnung aus den Gewässern

Die Extrakte aus den Gewässern werden gegebenenfalls über Amberlite XAD-7 konzentriert. Die Fraktionen, die organische Halogene, PCBs, Phenole etc. enthalten, werden entsprechend früher publizierter Verfahren gewonnen (Müller et al., Environm. Toxicol. Chem. 14,1203-1208,1995).

### 2.3 Nachweis der Expression von 14-3-3 Protein auf mRNA-Ebene

Aufgrund der Tatsache, daß weite Bereiche der verschiedenen 14-3-3 Isoformen sehr konserviert sind (FIG. 2), können degenerierte Primer zur Identifikation und zur Quantifizierung der mRNA eingesetzt werden. Für die quantitative PCR-Analyse wurde von den Erfindern bereits ein Primer, gerichtet gegen aa47-aa53 ; VAYKNWG, 5'-GTKGCCTACAARAAYGTGGT-3' [K = G/T, R = A/G und Y = C/T ; Vorwärtsprimer] mit Erfolg angewandt.

Für eine Quantifizierung der mRNA für 14-3-3 Protein mittels des NorthernBlotting-Verfahrens werden Gensonden hergestellt, die von den konservierten Anfangsbereichen [aa 45-aa 57] bis zu den konservierten Endbereichen [aa 214aa 234] des 14-3-3 Proteins reichen (FIG. 2). Als Methodik wird die PCR-Klonierung eingesetzt. Die erhaltenen 500 bp langen Sonden eignen sich erfahrungsgemäß gut für Northem-Blotting-Untersuchungen.

Die quantitative Auswertung der Signale kann zum Beispiel mit Hilfe des Chemilumineszenz-Verfahrens (Stanley und Kricka, Bioluminescence and chemiluminescence : current status. John Wiley & Sons, New York, 1990) in Verbindung mit einem Phospholmager (z. B. GS-525 Molecular Imager der Firma Bio-Rad) erfolgen ; Dinatrium 2-Chlor-5-(4-methoxyspiro {1,2-dioxetan-3,2'-(5'-chlor)-tricyclo[3.3.1.13,7]decan}-4-yl)phenyl-phosphat (CDP) wird hierfür als Substrat angewandt.

### 2.4 Nachweis der Expression von 14-3-3 auf Protein-Ebene : Western-Blotting

Der Nachweis der Expression der 14-3-3 Proteine auf Protein-Ebene erfoigt mittels Antikörper. Entweder können kommerzielle (z. B. von der Firma Calbiochem/ Oncogene) oder gegen das rekombinante Invertebraten-Protein 14-3-3 aus *Geodia cydonium* gezogene Antikörper eingesetzt werden. Zur Quantifizierung der 14-3-3 Proteine werden Western-Blots mit Extrakten des Bioindikators hergestellt. Die Signale können wieder unter Anwendung des Chemilumineszenz-Verfahrens quantifiziert werden. Die Gewebeextrakte können z. B. durch Homogenisierung in einem Phosphat-Puffer, enthaltend 1 mM EDTA und 1 mM Phenylmethylsulfonylfluorid, erhalten werden.

### Beispiel 1. Anwendung von 14-3-3 Protein als Biomarker für PCB: Nachweis mittels Northern- und Westem-Blotting

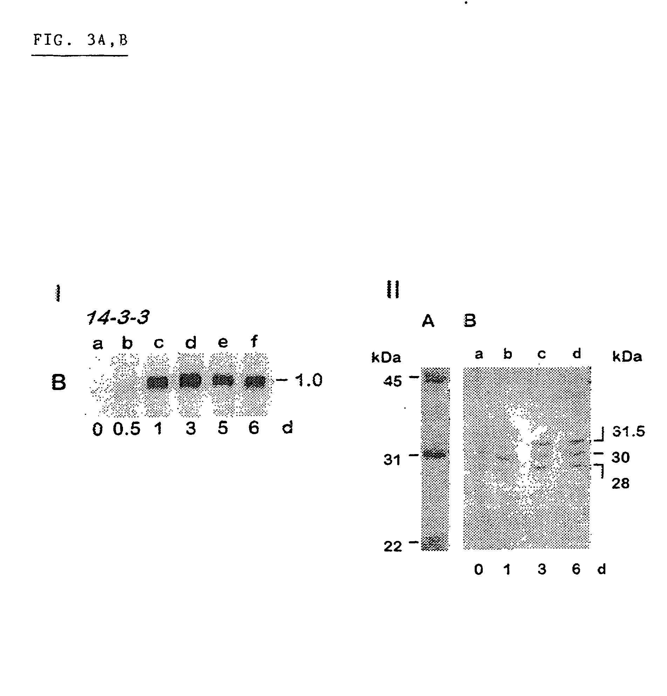

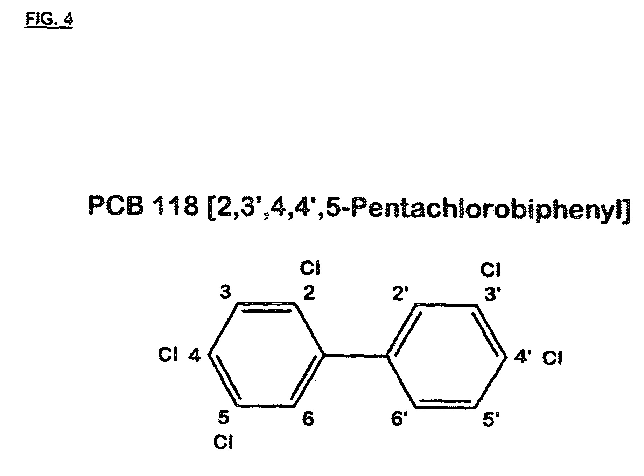

Die 14-3-3 cDNA des Meeresschwammes *Geodia cydonium* wurde als Gensonde zum Nachweis der Expression des 14-3-3 Proteins auf der mRNA-Ebene und die entsprechenden Antikörper zur Bestimmung der Proteinmenge eingesetzt; FIG. 3A und B. Bei den durchgeführten Hälterungsuntersuchungen wurde als Modell-PCB das PCB 118 (FIG. 4) verwendet.

### Durchführung :

(a) Exposition von *G. cydonium* gegenüber PCB 118:
   1 ml PCB 118 (0.1 mg/ml in Maisöl) wurde in 40 g Schwammgewebe injiziert. Die Proben wurden in Seewasser für bis zu 6 Tage in 20 1 Aquarien bei 17 C unter kontinuierlicher Belüftung inkubiert; das Wasser wurde einmal am zweiten Tag gewechselt. Aliquote Teile des Gewebes (ungefähr je 200 mg) wurden zum Zeitpunkt Null oder nach einer Inkubationszeit von 0,5,1,3,5 und 6 Tagen entnommen. Diese aliquoten Teile wurden sofort in flüssigem Stickstoff eingefroren und bei-80 C aufbewahrt.
(b) Extraktion: Extrakte zur Bestimmung der Menge an 14-3-3 Protein wurden durch Mörsern der gefrorenen Gewebeproben in dem dreifachen Volumen an Phosphat-Puffer unter Zusatz von 1 mM EDTA und 1 mM Phenylmethylsulfonylfluorid gewonnen. RNA wurde aus dem in flüssigem Stickstoff pulverisierten Schwammgewebe mit Hilfe des TRlzol Reagenz (GibcoBRL) nach den Angaben des Herstellers extrahiert.

Die Hälterungsuntersuchungen mit *G. cydonium* zeigten, daß PCB 118 die Expression des 14-3-3-Gens von nicht nachweisbar auf sehr hohe Werte induziert (FIG. 3A und B).

Vergleichbar starke und eindeutige Effekte konnten bisher noch mit keinem anderen Biomarker für PCB bei Invertebraten nachgewiesen werden.

Weiterhin ist bemerkenswert und wichtig für eine potentielle Anwendung als Biomarker bei anderen aquatischen Invertebraten, daß sowohl die Antikörper gegen das 14-3-3 Protein des Schwammes als auch die Gensonde von *G. cydonium* mit den homologen Proteinen/RNAs höherer Invertebraten und Vertebraten kreuzreagieren/hybridisiert.

### Beispiel 2. Anwendung von 14-3-3 Protein als Biomarker für (Xeno) Östrogene : Nachweis mittels Northern-Blotting

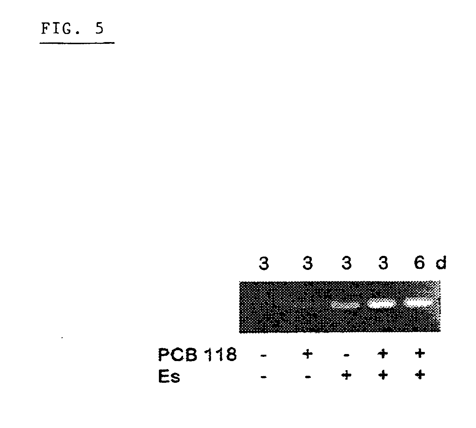

Durch Northern-Blotting konnte gezeigt werden, daß PCB sowie 17ss-Östradiol die Expression von 14-3-3 in niederen Invertebraten stark induziert (FIG. 5). Weiter konnte nachgewiesen werden, daß nach Kombination beider Substanzen eine Potenzierung der Expression der 14-3-3 mRNA eintrat. In Abwesenheit beider Agenzien läßt sich 14-3-3 mRNA nicht detektieren. Während das Niveau der 14-3-3 mRNA bei Applikation von PCB 118 bei 30% und von 17ss-Östradiol bei 100% [Bezugswert] lag, induzierten beide Chemikalien die Expression von 14-3-3 auf 550%. Die Induktion ist nach 3 Tagen abgeschlossen.

### 2.5 Nachweis der Expression von 14-3-3 Protein mittels eines ELISA-Verfahrens

### 2.5.1 Präparation der ELISA-Mikrotiterplatten

14-3-3 ist ein spezifisch Phosphoserin-bindendes Protein ; das 14-3-3 Bindemotiv ist identifiziert (Muslin et al., Cell 84,889-897,1996). In dem von uns entwickelten ELISA haben wir das Peptid LPKINRSApSEPSLHR (pS = Phosphoserin), an welches das 14-3-3 Protein mit hoher Affinität bindet (Muslin et al., Cell 84,889-897, 1996), N-terminal über zwei Alanine als Spacer mit einem Cystein verknüpft.

Variante 1 : Hierbei wurde das chemisch synthetisierte Peptid CAALPKINRSApSEPSLHR durch Reaktion der Sulfhydrylgruppe des N-terminalen Cysteins an Reacti-BindTM Maleimid-aktivierte Mikrotiterplatten (Kapazität: 100-150 pmol SH-enthaltende Peptide pro Well ; Firma Pierce, Rockford, IL, USA) kovalent gebunden. Vorgegangen wurde hierbei nach den Instruktionen des Herstellers. Überschüssige Maleimidgruppen auf den Platten wurden durch einstündige Inkubation der Platten mit einer Cysteinlösung (10 µg/ml) blockiert (alternativ : 3stündige Inkubation mit einer 5% igen Rinderserumalbumin-Lösung).

Vor der Kopplung an die Platten wurde die Peptidlösung mit Ellman's Reagenz auf die Gegenwart von SH-Gruppen getestet. Gegebenenfalls (im Falle einer Oxidation der SH-Gruppen unter Ausbildung von Disulfidbindungen) wurde das Peptid mit dem ReduceImm Kit von der Firma Pierce (siehe oben) behandelt.

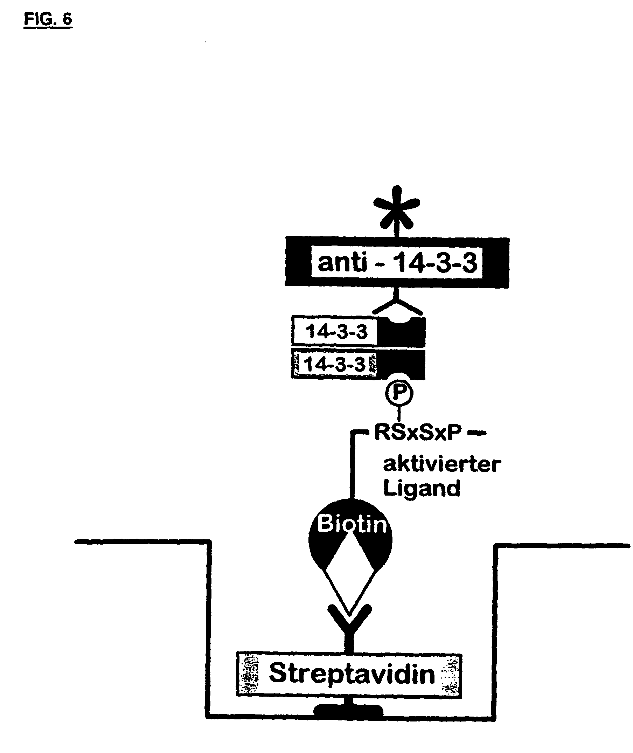

Variante 2 : Bei diesem alternativen Verfahren wurde das chemisch synthetisierte Peptid CAALPKINRSApSEPSLHR durch Reaktion der Sulfhydrylgruppe des N-terminalen Cysteins an das EZ-LinkTM Biotinylierungs-Reagens Biotin-BMCC {1- Biotinamido-4- [4'-(maleimidomethyl)-cyclohexancarboxyamido] butan} von der Firma Pierce (siehe oben) kovalent gebunden. Die Bindung der biotinylierten Proteine an die Mikrotiterplatten erfolgt in diesem Fall über Streptavidin (Verwendung von Streptavidin-beschichteten Mikrotiterplatten; FIG. 6).

### 2.5.2 Durchführung des ELISA

Die Durchführung erfolgt in folgenden Schritten :

### Variante 1 :

1 a. Inkubation der Streptavidin-beschichteten Mikrotiterplatten mit dem Biotingekoppelten Peptid.
2a. Zugabe von 100 µl Extrakt aus den untersuchten Tiergeweben in die Wells nach Bindung des Peptids über Biotin und Streptavidin.

Oder : 1a. Inkubation der Extrakte aus den untersuchten Tiergeweben mit dem Biotingekoppelten Peptid.
2a. Zugabe von 100 µl der Inkubationsmischungen nach Bindung des Biotin-Peptids an 14-3-3 Protein in die Wells.
3. Waschen.
4. Quantifizierung der Peptid-14-3-3 Protein-Komplexe über markierte Antikörper.
   Als Markierung kann zum Beispiel die alkalische Phosphatase verwendet werden; als Substrat zum Nachweis dient dann 5-Brom-4-Chlor-3-Indolylphosphat/Nitroblau Tetrazolium.
5. Detektion mittels eines ELISA-Platten-Readers.

### Variante 2:

1. Zugabe von 100 µl Extrakt in die Wells der Mikrotiterplatten mit dem gekoppelten Peptid und Inkubation.
2. Waschen.
3. Quantifizierung der Peptid-14-3-3 Protein-Komplexe über markierte Antikörper (siehe Variante 1).
4. Detektion mittels eines ELISA-Platten-Readers.

### Beispiel 3. Quantifizierung von 14-3-3 Protein in Klieschen nach Exposition gegenüber PCBs und Cadmium: Nachweis mittels des ELISA-Verfahrens

Die in Tabelle 1 angegebenen Mengen an PCB77, PCB118 und PCB153 wurden nach Auflösung in Maisöl in Klieschen (*Limanda limanda*) injiziert. Die Injektion der angegebenen Mengen an Cadmium (Tabelle 2) erfolgte nach Lösung in PBS. Nach den angegebenen Zeiträumen wurde den Tieren die Leber entnommen und sofort eingefroren. Die Bestimmung des Gehalts an 14-3-3 Protein in den Lebern erfolgte nach Homogenisation durch Mörsern der gefrorenen Gewebeproben in dem dreifachen Volumen an Phosphat-Puffer mit 1 mM EDTA und 1 mM Phenylmethylsulfonylfluorid.

**Tabelle 1:**

| Injektion [i. p.] von 0.03 mg PCB pro kg *Limanda limanda* (Kliesche Fisch). Nach 5 Tagen wurden die Lebern entnommen und die Menge an 14-3-3 Protein in dem angegebenen ELISA bestimmt. Die optischen Dichten sind in OD Einheiten angegeben. | |
|---|---|
| Verbindung | 14-3-3 Protein (OD-Einheit) |
| PCB77 | 0, 97 |
| PCB 118 | 0, 74 |
| PCB153 | 0,81 |
| Kontrolle | 0, 12 |

**Tabelle 2:**

| Injektion [i. p.] von Cadmium in *Limanda limanda* (Kliesche-Fisch). Nach 5 Tagen wurden die Lebern entnommen und die Menge an 14-3-3 Protein in dem angegebenen ELISA bestimmt. Die optischen Dichten sind in OD-Einheiten angegeben. | |
|---|---|
| Cadmium (mg/kg) | 14-3-3 Protein (OD-Einheit) |
| 0,01 | 0, 30 |
| 0,03 | 0, 75 |
| 0,1 | 0, 98 |
| Kontrolle | 0,12 |

### 3. Erläuterungen zu den Abbildungen : Im nachfolgenden sind die erläuternden Legenden zu den auf den Seiten 1/6-6/6 abgebildeten Grafiken aufgeführt:

FIG. 1 zeigt eine schematische Darstellung der Rolle von 14-3-3, einem "lenkenden" Chaperon, und HSP70, einem Protein-Faltungs-Chaperon. Im Text ist ausgeführt, daß durch PCB 118 eine Induktion des Gens für 14-3-3 -untersucht an dem Meeresschwamm *Geodia cydonium-* stattfindet Nach Dimerisierung bindet 14-3-3 an das Bindungsmotiv, RSxSxP [nach erfolgter Phosphorylierung] von Schlüsselmolekülen, die in intrazelluläre Signaltransduktionskaskaden oder in den Apoptose-Prozess involviert sind und verhindert deren Translokation in dasjenige Zellkompartiment, in dem die betreffende Funktion-wie Initiation einer Genexpression oder auch Apoptose-eingeleitet werden soll. Im Gegensatz dazu bindet HSP70 an Zielproteine und ermöglicht deren funktionelle Faltungen.

FIG. 2 zeigt einen Vergleich des abgeleiteten 14-3-3 Proteins von *G. cydonium* [GEODIA-ge] mit folgenden Isoformen : y der Ratte [RAT-gamma], rl vom Mensch [HOMO-eta], C vom Schaf [SHEEP-zeta], dem 14-3-3-verwandten Polypeptid von Xenopus laevis [XENLA-D2,214097], ss der Ratte [RAT-beta], 0 der Ratte [RATtheta], Drosophila melanogaster 14-3-3 Protein [DROME-LP], dem 14-3-3 Protein von Caenorhabditis elegans [CAEL-cds4] und a vom Mensch [HOMO-sigma]. Konservierte Aminosäuren in allen Sequenzen sind invertiert dargestellt ; solche die in mehr als fünf Sequenzen vorkommen, sind schattiert unterlegt. Die Sequenz, die zur Antikörperproduktion diente, ist gekennzeichnet (*****).

FIG. 3 zeigt den Effekt von PCB 118 auf die Expression der mRNA von 14-3-3 nach Inkubation der Tiere mit 2 µg PCB 118/g Feuchtgewicht für 0,5 bis 6 Tage. Die Untersuchungen wurden mittels Northern-Blotting [14-3-3-Sonde] (A) bzw. Western Blotting [14-3-3-Antikörper] (B) durchgeführt.

In FIG. 4 ist die Strukturformel von PCB 118 dargestellt.

In FIG. 5 ist der Effekt von PCB 118 (2 ug/g) und 17ss-Östradiol (20 ng/g) auf die Expression des 14-3-3 Gens gezeigt. Die beiden Chemikalien wurden entweder alleine oder in Kombination den Versuchstieren (*G. cydonium*) zugesetzt. Die Inkubationszeit wurde auf 3 bzw. 6 Tage begrenzt.

FIG. 6 zeigt eine schematische Darstellung des Prinzips des entwickelten ELISA Verfahrens zur quantitativen Bestimmung von 14-3-3 (Variante 2).

## Patentansprüche

1. In vitro-Verfahren zum Nachweis von natürlichen und anthropogenen Umweltchemikalien in Gewässern oder im Boden, **dadurch gekennzeichnet, daß** 14-3-3 Proteine als Biomarker in nicht-menschlichen Bioindikatororganismen, die dem zu untersuchenden Gewässer oder Boden ausgesetzt werden, bestimmt werden.

2. Verfahren nach Anspruch 1, wobei als Bioindikatororganismen nicht-menschliche Vertebraten oder Invertebraten verwendet werden.

3. Verfahren nach Anspruch 1 oder 2, wobei die Bioindikatororganismen ausgewählt sind aus der Gruppe bestehend aus *Corbicula fluminea, Limanda limanda, Suberites domuncula* und *Geodia cydonium*.

4. Verfahren nach einem der Ansprüche 1 bis 3, wobei die natürlichen und anthropogenen Umweltchemikalien ausgewählt sind aus polychlorierten Biphenylen und/oder (Xeno) Östrogenen.

5. Verfahren nach einem der Ansprüche 1 bis 4, wobei das Verfahren weiterhin ein Aufkonzentrieren der zu untersuchenden Probe bei potentiell schwachen Effekten, z. B. bei Flusswässern, umfaßt.

6. Verfahren nach einem der Ansprüche 1 bis 5, wobei das Verfahren weiterhin eine Quantifizierung der Expression von 14-3-3 Protein umfaßt.

7. Verfahren nach einem der Ansprüche 1 bis 6, wobei die Bestimmung und/oder die Quantifizierung der Expression des 14-3-3 Proteins mittels degenerierter Primer, Northern-Blotting, Western-Blotting und/oder mittels eines ELISA-Verfahrens erfolgt.

8. Verfahren nach Anspruch 7, wobei das ELISA-Verfahren **dadurch gekennzeichnet ist, daß** zur spezifischen Bindung der 14-3-3 Proteine Mikrotiterplatten verwendet werden, die mit einem Peptid, welches ein Bindemotiv des 14-3-3 Proteins besitzt, beschichtet sind.

9. Verfahren nach Anspruch 8, wobei das Peptid die Sequenz CAALPKINRSApSEPSLHR (pS Phosphoserin) aufweist.

10. Laborverfahren zum Biomonitoring, z. B. zur Überwachung kommunaler und industrieller Klärwerkszu- und abläufe, Flusswässer oder Meerwasser, umfassend ein Verfahren nach einem der Ansprüche 1 bis 9.

## Claims

1. *In vitro* method for the detection of natural and anthropogenic environmental chemicals in waters or soil, **characterised in that** 14-3-3 proteins are determined as biomarker in bioindicative organisms that are exposed to the waters or soil to be examined.

2. Method according to claim 1, wherein non-human vertebrates or invertebrates are used as bioindicative organisms.

3. Method according to claim 1 or 2, wherein said bioindicative organisms are selected from the group consisting of *Corbicula fluminea, Limanda limanda, Suberites domuncula,* and *Geodia cydonium*.

4. Method according to any of claims 1 to 3, wherein said natural and anthropogenic environmental chemicals are selected from polychlorinated biphenyls and/or (xeno)estrogenes.

5. Method according to any of claims 1 to 4, wherein said method furthermore comprises concentrating of the sample to be examined in case of potentially weak effects, e.g. in waters of rivers.

6. Method according to any of claims 1 to 5, wherein said method furthermore comprises a quantification of the expression of 14-3-3 protein.

7. Method according to any of claims 1 to 6, wherein said detecting and/or the quantification of the expression of the 14-3-3 proteins takes place by means of degenerated primers, Northern-blotting, Western-blotting and/or by means of an ELISA-method.

8. Method according to claim 7, wherein said ELISA-method is **characterised in that** for a specific binding of the 14-3-3 proteins microtiter plates are used that are coated with a peptide that has a binding motif of the 14-3-3 protein.

9. Method according to claim 8, wherein said peptide has the sequence CAALPKINRSApSEPSLHR (pS phosphoserine).

10. Laboratory method for biomonitoring, e.g. for controlling communal and industrial sewage plant in- and outlets, waters of rivers or marine water, comprising a method according to any of claims 1 to 9.

## Revendications

1. Procédé in vitro de détection de produits chimiques naturels et anthropogènes susceptibles de polluer l'environnement, dans les eaux ou dans le sol, **caractérisé en ce que** des protéines 14-3-3 sont déterminés comme biomarqueurs dans des organismes bioindicateurs non humains qui sont exposés aux eaux ou au sol à étudier.

2. Procédé selon la revendication 1, dans lequel des vertébrés non humains ou des invertébrés sont utilisés comme organismes bioindicateurs.

3. Procédé selon la revendication 1 ou 2, dans lequel les organismes bioindicateurs sont choisis dans le groupe constitué de *Corbicula fluminea, Limanda limanda, Suberites domuncula* et *Geodia cydonium*.

4. Procédé selon l'une quelconque des revendications 1 à 3, dans lequel les produits chimiques naturels et anthropogènes susceptibles de polluer l'environnement sont choisis parmi les biphényles polychlorés et/ou les (xéno) oestrogènes.

5. Procédé selon l'une quelconque des revendications 1 à 4, dans lequel le procédé comporte en outre une concentration de l'échantillon en cas d'effets potentiellement faibles, par exemple pour les eaux fluviales.

6. Procédé selon l'une quelconque des revendications 1 à 5, dans lequel le procédé comporte en outre une quantification de l'expression de la protéine 14-3-3.

7. Procédé selon l'une quelconque des revendications 1 à 6, dans lequel la détermination et/ou la quantification de l'expression de la protéine 14-3-3 s'effectue au moyen de « primer » dégénérés, des procédés Northern-Blotting, Western-Blotting et/ou au moyen d'un procédé ELISA.

8. Procédé selon la revendication 7, dans lequel le procédé ELISA est **caractérisé en ce qu'**on utilise pour la liaison spécifique des protéines 14-3-3 des plaques de microtitrage qui sont enduites d'un peptide, lequel possède un motif de liaison de la protéine 14-3-3.

9. Procédé selon la revendication 8, dans lequel le peptide présente la séquence CAALPKINRSApSEPSLHR (pS phosphosérine).

10. Procédé de biosurveillance au laboratoire, par exemple pour la surveillance des affluents et des effluents communaux et industriels des installations d'épuration, celle des eaux fluviales ou des eaux de mer, comprenant un procédé selon les revendications 1 à 9.
